# EUROPEAN PATENT SPECIFICATION

(11) **EP 3 411 011 B1**
(45) Date of publication and mention of the grant of the patent: **18.09.2024**
(21) Application number: 17748282.5
(22) Date of filing: 03.02.2017
(51) Int. Cl.: A61K 8/60, A61K 8/36, A61K 8/67, A61Q 17/00, A61Q 19/00, A61Q 19/08, A61K 8/99, A61K 31/714, A61K 35/74, C12Q 1/04, G01N 33/82, A61P 17/10

(54) **COMPOSITIONS AND METHODS FOR PROMOTING SKIN HEALTH**
ZUSAMMENSETZUNGEN UND VERFAHREN ZUR FÖRDERUNG DER HAUTGESUNDHEIT
COMPOSITIONS ET MÉTHODES FAVORISANT LA SANTÉ DE LA PEAU

(30) Priority: 05.02.2016 US 201662291665 P
(43) Date of publication of application: 12.12.2018
(73) Proprietor: The Regents of the University of California, Oakland, CA 94607-5200 (US)
(72) Inventor: LI, Huiying, Los Angeles CA 90077 (US); JOHNSON, Tremylla, Los Angeles CA 90066 (US); KANG, Dezhi, Alhambra CA 91801 (US); BARNARD, Emma, Los Angeles CA 90066 (US)
(74) Representative: Müller-Boré & Partner Patentanwälte PartG mbB
(86) International application number: PCT/US2017/016526
(87) International publication number: WO 2017/136738

(56) References cited:
- WO-A1-2015/171899
- WO-A1-2015/171899
- WO-A1-2017/184992
- WO-A1-2017/185016
- WO-A1-2018/089337
- US-A- 5 981 473
- US-A1- 2003 147 930
- US-A1- 2015 086 581
- SOREL FITZ-GIBBON ET AL: "Propionibacterium acnes Strain Populations in the Human Skin Microbiome Associated with Acne", JOURNAL OF INVESTIGATIVE DERMATOLOGY, vol. 133, no. 9, 21 January 2013 (2013-01-21), pages 2152 - 2160, XP055166702, ISSN: 0022-202X, DOI: 10.1038/jid.2013.21
- FITZ-GIBBON, SOREL ET AL.: "Propionibacterium acnes strain populations in the human skin microbiome associated with acne", JOURNAL OF INVESTIGATIVE DERMATOLOGY, vol. 133, 28 February 2013 (2013-02-28), pages 2152 - 2160, XP055166702
- JOHNSON, TREMYLLA ET AL.: "Strain-level differences in porphyrin production and regulation in Propionibacterium acnes elucidate disease associations", MSPHERE, vol. 1, no. 1, 10 February 2016 (2016-02-10), pages 1 - 12, XP055432883

## Description

### BACKGROUND

*Propionibacterium acnes* is a major commensal bacterium residing on the human skin. It plays important roles in maintaining skin health, but has also been implicated in the pathogenesis of several diseases and infections, including sarcoidosis, SAPHO syndrome, endodontic lesions, eye infections, prosthetic joint infections, prostate cancer, and acne vulgaris (commonly called acne). Acne is the most common skin disease affecting more than 80% of adolescents and young adults worldwide. Despite the clinical importance of the disease, the etiology of acne is not yet clear. *P. acnes* dominates the skin of both acne patients and healthy individuals, and thus its role in acne pathogenesis has not been well understood.

### SUMMARY

In some aspects, the invention relates to a topical composition comprising an RT1 strain of *Propionibacterium acnes* selected from the group consisting of HL050PA2 and HL025PA1 or an RT2 strain of *Propionibacterium acnes* selected from the group consisting of HLOO1PA1, HL103PA1 and HL106PA1 for use as a medicament.

In some aspects, the invention relates to a topical composition comprising an RT1 strain of *Propionibacterium acnes* selected from the group consisting of HL050PA2 and HL025PA1 or an RT2 strain of *Propionibacterium acnes* selected from the group consisting of HL001PA1, HL103PA1 and HL106PA1 for use in treating a skin condition selected from the group consisting of acne, rosacea, and porphyria cutanea tarda.

In some aspects, the invention relates to a cosmetic method of preventing skin aging in a subject comprising administering a topical composition comprising an RT1 strain of *Propionibacterium acnes* selected from the group consisting of HL050PA2 and HL025PA1 or an RT2 strain of *Propionibacterium acnes* selected from the group consisting of HL001PA1, HL103PA1 and HL106PA1 to the subject.

Further embodiments of the invention are defined in the appended claims.

HL001PA1 is a clonal complex 72 (CC72) strain based on the Belfast MLST₈ classification scheme; and HL001PA1 is a clonal complex 60 (CC60) strain based on the Aarhus MLST₉ classification scheme.

In some aspects, the disclosure relates to a method for determining the risk that a subject will develop vitamin B₁₂-induced acne, comprising measuring the porphyrin production of *P. acnes* obtained from the subject.

In some aspects, the disclosure relates to a method for determining a risk that a subject will develop vitamin B₁₂-induced acne, comprising identifying one or more strains of *P. acnes* obtained from the subject, and diagnosing the subject with an elevated risk of developing vitamin B₁₂-induced acne if one or more of the strains of *P. acnes* is sensitive to vitamin B₁₂. In some aspects, the disclosure relates to a method for determining a risk that a subject will develop vitamin B₁₂-induced acne, comprising identifying one or more strains of *P*. *acnes* obtained from the subject, and diagnosing the subject with an elevated risk of developing vitamin B₁₂-induced acne if one or more of the strains of *P*. *acnes* produces elevated levels of porphyrins.

In some aspects, the disclosure relates to a topical composition, comprising a nucleic acid encoding *deoR.* In other aspects, the disclosure relates to a composition formulated for oral delivery comprising a nucleic acid encoding *deoR.*

In some aspects, the disclosure relates to a method for treating a skin condition in a subject, comprising: measuring the porphyrin production of *P*. *acnes* obtained from the subject; and treating the subject with photodynamic therapy if the porphyrin production is above a threshold value. In some embodiments, the disclosure relates to a method for treating a skin condition in a subject, comprising determining the identity of a strain of *P*. *acnes* obtained from the subject; and treating the subject with photodynamic therapy if the strain of *P*. *acnes* produces elevated porphyrin levels.

In some aspects, the disclosure relates to compositions and methods for treating a skin condition in a subject, comprising administering to the subject a composition comprising *P*. *acnes,* wherein the *P*. *acnes* comprises a nucleic acid encoding *deoR.* The composition may be formulated for topical delivery. The compositions may be formulated for oral delivery.

In some aspects, provided herein are methods of reducing the amount of porphyrins on the skin of a subject by administering a composition comprising one or more strains of *Propionibacterium acnes* to the subject. The subject may have acne, skin aging, rosacea, or porphyria cutanea tarda.

In some aspects, provided herein are compositions formulated for oral delivery for treating a skin condition or maintaining skin health, comprising an RT1, RT2, RT3, RT6, RT8, RT16, or Type III strain of *Propionibacterium acnes.* In other aspects, provided herein are compositions (e.g., oral or topical compositions) for treating a skin condition or maintaining skin health, comprising a strain of *Propionibacterium acnes,* wherein the strain of *Propionibacterium acnes* comprises a nucleic acid encoding *deoR.* A strain of *P. acnes* may be selected from HL042PA3, HL050PA2, HL025PA1, HL001PA1, HL103PA1, HL106PA1, HL110PA3, HL025PA2, HL046PA1, HL110PA2, HL053PA2, HL059PA1, HL201PA1, 17A, or 20C.

### BRIEF DESCRIPTION OF THE FIGURES

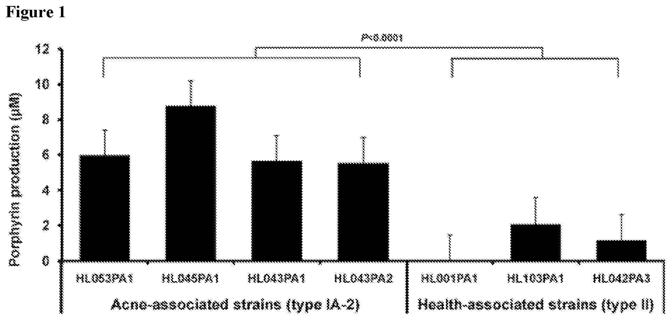

**Figure 1****. Acne-associated type IA-2 *P. acnes* strains produced significantly more porphyrins than health-associated type II strains.** Each bar represents the porphyrins produced by each strain normalized by the bacterial culture density. Shown is the mean of the data obtained from at least three independent experiments with at least three replicates each. Error bars represent standard error.

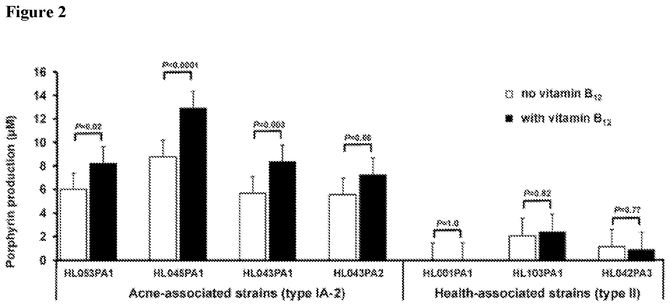

**Figure 2****. Vitamin B₁₂ supplementation significantly increased porphyrin production in acne-associated type IA-2 strains, but not in health-associated type II strains.** *P. acnes* strains were cultured in media with (black bars) or without (white bars) the addition of 10 µg/mL vitamin B₁₂. Each bar represents the porphyrins produced by each strain normalized by the bacterial culture density. Shown is the mean of the data obtained from at least three independent experiments with at least three replicates each. Error bars represent standard error.

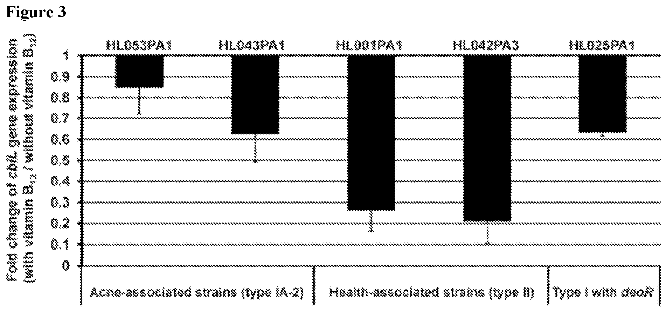

**Figure 3****. Vitamin B₁₂ supplementation repressed the expression of a vitamin B₁₂ biosynthesis gene *cbiL.*** The expression level of *cbiL* was quantified by qRT-PCR from *P*. *acnes* strains cultured with or without the addition of 10 µg/mL vitamin B₁₂. Strains of types IA-2 and II and an RT1 strain encoding *deoR,* HL025PA1, are shown. Each bar represents the fold change in gene expression of *cbiL* in cultures with vitamin B₁₂ supplementation compared to cultures without supplementation. Shown is the mean of the data obtained from three independent experiments with three replicates each. Error bars represent standard deviation.

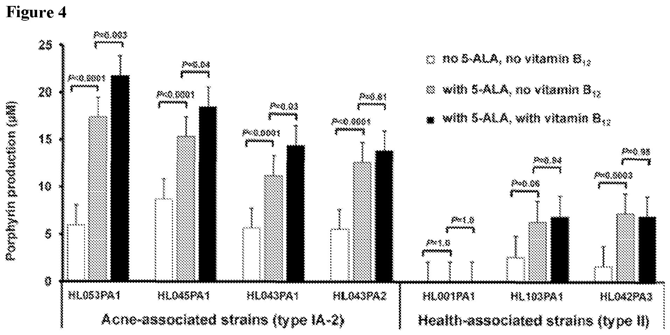

**Figure 4**. 5-ALA increased porphyrin production, which was further enhanced by **vitamin B₁₂ supplementation in acne-associated type IA-2 strains.** *P. acnes* strains were cultured in media with (grey bars) or without (white bars) substrate 5-ALA (0.1 mg/mL), or with both 5-ALA and vitamin B₁₂ (10 µg/mL) added (black bars). 5-ALA significantly increased porphyrin production in acne-associated type IA-2 strains (*P* <0.0001) and in health-associated type II strains (*P*<0.06, except HL001PA1). Vitamin B₁₂ supplementation further increased porphyrin production in the presence of 5-ALA in acne-associated type IA-2 strains, but not in health-associated type II strains. Each bar represents the porphyrins produced by each strain normalized by the bacterial culture density. Shown is the mean of the data obtained from at least three independent experiments with at least three replicates each. Error bars represent standard error.

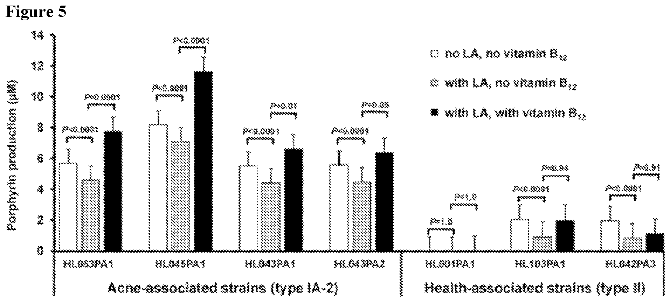

**Figure 5****. Small molecule inhibitor reduced porphyrin production in *P. acnes* and its inhibition was counteracted by vitamin B₁₂ supplementation in acne-associated type IA-2 strains.** *P. acnes* strains were cultured in media with (grey bars) or without (white bars) inhibitor LA (0.1 mg/mL), or with both LA and vitamin B₁₂ (10 µg/mL) added (black bars). LA significantly reduced porphyrin biosynthesis in all strains except HL001PA1 (*P* ≤0.0001). Vitamin B₁₂ supplementation counteracted the inhibition of porphyrin biosynthesis by LA in acne-associated type IA-2 strains, but not in health-associated type II strains. Each bar represents the porphyrins produced by each strain normalized by the bacterial culture density. Shown is the mean of the data obtained from at least three independent experiments with at least three replicates each. Error bars represent standard error.

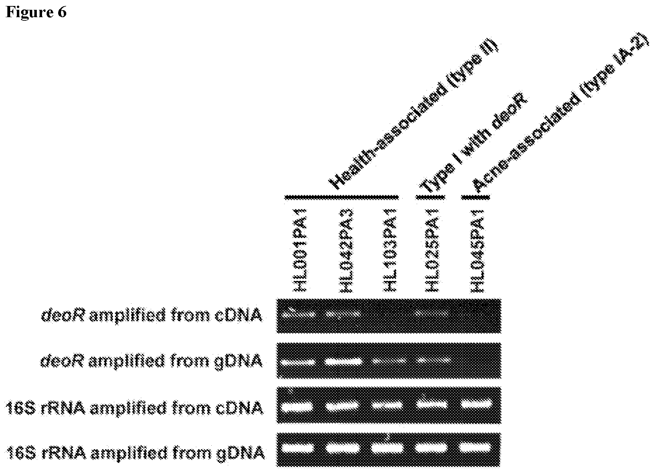

**Figure 6****. Health-associated strains encode and expressed *deoR,* a repressor gene in the porphyrin biosynthesis operon.** *deoR* amplification from the cDNA and genomic DNA (gDNA) samples of multiple strains is shown in the gel image. 16S rRNA gene was used as a positive control. HL045PA1, which is an acne-associated type IA-2 strain and does not encode *deoR,* is shown as a negative control.

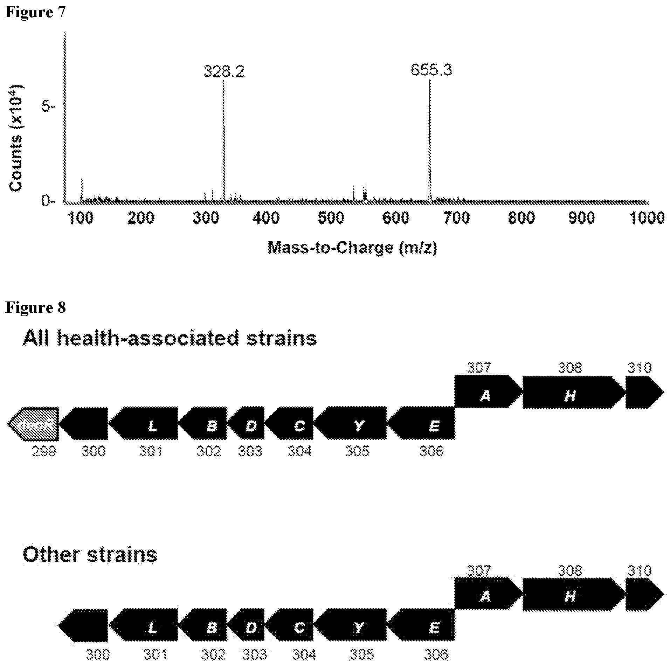

**Figure 7****. Coproporphyrin was the dominant porphyrin isoform produced by *P. acnes.*** Porphyrins secreted by *P. acnes* have a mass spectrum characteristic of the monoisotopic coproporphyrin isoform ([M + H]⁺ = 655.3). The doubly charged parent ion was also observed ([M + 2H] ²⁺ = 328.2).
**Figure 8****. Porphyrin (*Hem*) gene cluster in *P. acnes* strains.** All health-associated type II strains harbor a *deoR* transcription repressor (PPA0299, in green) in the porphyrin gene cluster. The number below each box represents the gene ID based on the gene annotation in KPA171202. The letters in black boxes indicate the names of the *hem* genes, which are porphyrin biosynthesis genes. PPA0300 and PPA0310 were not assigned a *hem* gene name.

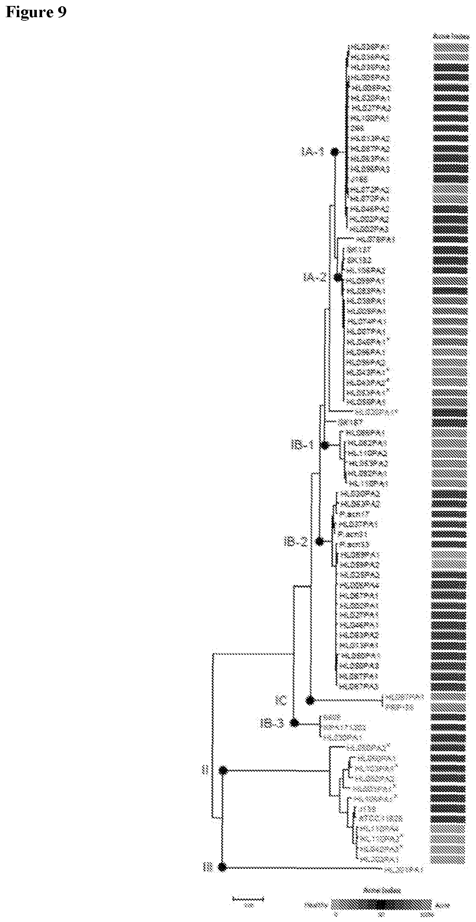

**Figure 9****. Presence of *deoR* in *P. acnes* lineages.** *P. acnes* strains encoding *deoR* are colored in green an include each type IC, IB-3, II, and III strain. Additionally, strain HL025PA1 of type IA-2 *P. acnes* encodes *deoR,* but no other type IA-1, IA-2, IB-1, or IB-2 strain was found to encode a *deoR* gene. Asterisks denote the strains tested in the Examples section.

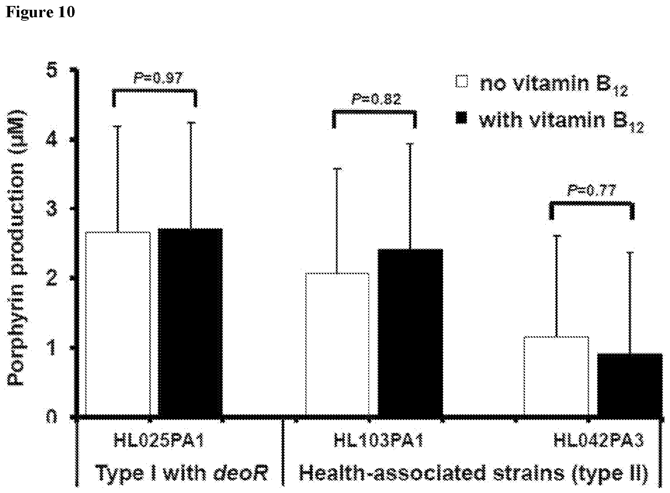

**Figure 10****. Similar to health-associated type II strains, HL025PA1 produced a low level of porphyrins and did not respond to vitamin B₁₂ supplementation.** HL025PA1 was cultured in the media with (black bars) or without (white bars) vitamin B₁₂ (10 µg/mL). Health-associated type II strains, HL103PA1 and HL042PA3, are shown for comparison. Each bar represents the porphyrins produced by each strain normalized by the bacterial culture density. Shown is the mean of the data obtained from three independent experiments with three replicates each. Error bars represent standard error.

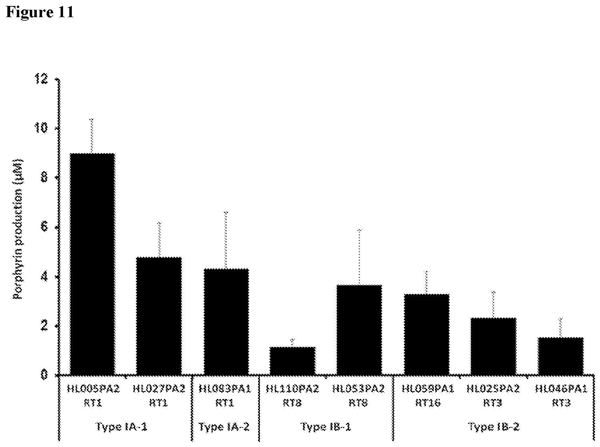

**Figure 11****. Porphyrin production in type I *P*. *acnes* strains.** Types IA-1 and IA-2 strains produced the highest levels of porphyrins among the tested strains. RT8, RT3, and RT16 strains produced less porphyrins than types IA-1 and IA-2 strains, but more than RT2 and RT6 strains. Each bar represents the porphyrins produced by each strain normalized by the bacterial culture density. Shown is the mean of the data obtained from at least two independent experiments with at least three replicates each. Error bars represent standard error.

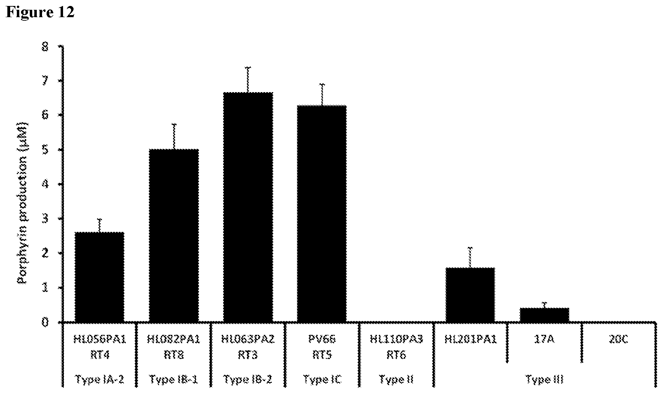

**Figure 12****. *P. acnes* type I strains produce significantly more porphyrins than type II and type III strains.** Each bar represents the porphyrins produced by each strain normalized by the bacterial culture density. Type I strains responded to vitamin B12 supplementation with increased porphyrin production. This was not observed in type II and type III strains (data not shown). Shown is the mean of the data obtained from at least one experiment with at least four replicates each. Error bars represent standard error.

### DETAILED DESCRIPTION

Some aspects of the invention are based on the finding that vitamin B₁₂ supplementation increases *P. acnes* production of porphyrins, a group of pro-inflammatory metabolites important in acne development. The porphyrin production and regulation of multiple *P. acnes* strains from acne- and health-associated lineages were compared. Acne-associated strains inherently produced significantly higher levels of porphyrins, which were further enhanced by vitamin B₁₂ supplementation. On the other hand, health-associated strains produced low levels of porphyrins and did not respond to vitamin B₁₂. Using small molecule substrates and inhibitors, porphyrin biosynthesis can be modulated at the metabolic level. Additionally, the repressor gene (*deoR*) of porphyrin biosynthesis was encoded in all health-associated strains, but not in acne-associated strains. The expression of *deoR* suggests additional regulation of porphyrin production at the transcriptional level in health-associated strains. These findings provide a molecular mechanism for the different contributions of *P. acnes* strains to skin health and disease, and further support the role of vitamin B₁₂ in acne pathogenesis. Specifically, the porphyrin biosynthesis pathway is a drug target for acne and related skin conditions, and health-associated strains of *P. acnes* may be used as probiotics in skin therapeutics.

The instant disclosure also suggests that health conditions of subjects with *P. acnes* that produce different levels of porphyrins might be treated differently. Vitamin B₁₂ modulation of porphyrin production is strain-specific, and suggests that the *P. acnes* strain composition of an individual's skin microbiota contributes to vitamin B₁₂-induced acne. Individuals harboring acne-associated strains are likely to be at increased risk for developing acne in response to high vitamin B₁₂ levels due to the ability of their skin bacteria to produce more porphyrins. On the other hand, individuals whose skin is dominated by health-associated strains may have lower porphyrin levels produced by the bacterium, leading to a reduced risk for acne development when supplemented with vitamin B₁₂.

### Definitions

As used herein the specification, "a" or "an" may mean one or more. As used herein in the claim(s), when used in conjunction with the word "comprising" the words "a" or "an" may mean one or more than one. As used herein "another" may mean at least a second or more.

The term "preventing" is art-recognized, and when used in relation to a condition such as a local recurrence (*e.g*., blemish), a disease such as acne, or an injury such as scarring, is well understood in the art, and includes administration of a composition which reduces the frequency of, or delays the onset of, symptoms of a medical condition in a subject relative to a subject which does not receive the composition. Thus, prevention of acne includes, for example, reducing the number of detectable blemishes (*e.g.,* lesions) in a population of patients receiving a prophylactic treatment relative to an untreated control population, and/or delaying the appearance of detectable blemishes in a treated population versus an untreated control population, *e.g*., by a statistically and/or clinically significant amount.

The terms "prophylactic treatment" and "therapeutic treatment" are art-recognized and include administration to a subject one or more of compositions. If a composition is administered prior to clinical manifestation of the unwanted condition (*e.g*., disease or other unwanted state of the host animal) then the treatment is prophylactic (*i.e.,* it protects the subject against developing the unwanted condition), whereas if it is administered after manifestation of the unwanted condition, the treatment is therapeutic (*i.e.,* it is intended to diminish, ameliorate, or stabilize the existing unwanted condition or side effects thereof).

The term "subject" refers to a mammal, including, but not limited to, a human or non-human mammal, such as a primate, bovine, equine, ovine, porcine, canine, lagomorph, feline, or rodent. In preferred embodiments, the subject is a human.

A "therapeutically effective amount" of a compound with respect to the subject method of treatment refers to an amount of the compound(s) in a preparation which, when administered as part of a desired dosage regimen (to a mammal, preferably a human) alleviates a symptom, ameliorates a condition, or slows the onset of disease conditions according to clinically acceptable standards for the disorder or condition to be treated or the cosmetic purpose, *e.g*., at a reasonable benefit/risk ratio applicable to any medical treatment.

As used herein, the term "treating" or "treatment" includes reversing, reducing, or arresting the symptoms, clinical signs, and underlying pathology of a condition in a manner to improve or stabilize a subject's condition.

### I. COMPOSITIONS

In some aspects, the invention relates to a topical composition for treating or preventing a condition in a subject. In some aspects, the invention relates to a topical composition for maintaining skin health. The composition is a topical composition. The condition may be a skin condition. The skin conditions may be acne, skin aging, rosacea, or porphyria cutanea tarda.

A composition comprises an RT1 strain of *Propionibacterium acnes* selected from the group consisting of HL050PA2 and HL025PA1 or an RT2 strain of *Propionibacterium acnes* selected from the group consisting of HL001PA1, HL103PA1 and HL106PA1.The composition may comprise more than one strain of *P. acnes.*

In preferred embodiments, the composition is substantially free from an RT4 strain of *P. acnes.* In preferred embodiments, the composition is substantially free from an RT5 strain of *P. acnes.* In preferred embodiments, the composition does not comprise HL045PA1, HL043PA1, HL043PA2, or HL053PA2. In some embodiments, the composition is substantially free from a type IA-1, IA-2, IB-1, and/or IC strain of *P. acnes.* In preferred embodiments, the composition is substantially free from a type IA-1, IA-2, and/or IC strain of *P. acnes.* In some embodiments, the composition is substantially free of strains that does not express, harbor, and/or encode *deoR.* In certain such embodiments, substantially free indicates that the composition does not comprise the indicated strain to an extent that compromises the therapeutic efficacy of the beneficial strains in the composition. In other such embodiments, a composition is substantially free of a strain if it comprises less than 10%, preferably less than 5%, and most preferably less than 1% of the indicated strain.

A composition may comprise a nucleic acid encoding *deoR* (*i.e.,* the nucleic acid may comprise the *deoR* nucleotide sequence). For example, the composition may comprise a vector and the vector may comprise the nucleic acid. The composition may comprise a strain of *P. acnes* and the strain of *P. acnes* may comprise the nucleic acid. The nucleic acid may be DNA or RNA.

The vector may be a phage or a plasmid. The vector may be a phage, and the phage may have a tropism for *Propionibacterium acnes, e.g.,* such that the phage can transform a *P. acnes* cell with the nucleic acid.

The nucleic acid may comprise a promoter, and the promoter and *deoR* may be operably linked, *e.g*., such that a cell comprising the nucleic acid can transcribe RNA from the nucleic acid.

The nucleic acid may be a recombinant nucleic acid. For example, *deoR* may be operably linked to a promoter other than a native *deoR* promoter. Similarly, *deoR* may be present in a vector that is not native to *P. acnes,* such as a vector comprising an origin of replication of a different species such as *E. coli* or a vector comprising a packaging signal for a bacteriophage. The nucleic acid may be the *P. acnes* genome and *deoR* may be randomly integrated into the genome.

A composition may comprise one or more strains of *P. acnes* that comprise the nucleic acid, *e.g.,* wherein the nucleic acid is the *P. acnes* genome, the nucleic acid is a plasmid, or the nucleic acid is RNA. A composition may comprise one or more strains of *P. acnes* that comprise a *deoR* protein product (*e.g.,* a transcriptional repressor protein). For example, such strains may express a *deoR* protein product. A composition may comprise one or more strains of *P. acnes* that encode or harbor a *deoR* gene. A composition may comprise one or more strains of *P. acnes* selected from RT1 or RT2 strains, and/or strains comprising a recombinant nucleic acid encoding *deoR.* A composition may comprise a clonal complex 72 (CC72) strain as determined by the Belfast MLST₈ classification scheme. A composition may comprise a clonal complex 60 (CC60) strain as determined by the Aarhus MLST₉ classification scheme.

A composition may further comprise levulinic acid, 4, 6-dioxoheptanoic acid, and/or isonicotinic acid hydrazide.

Compositions described herein may be used for oral administration to the gastrointestinal tract. The formulation for a composition (e.g., a probiotic composition) of the present invention may also include other probiotic agents or nutrients which promote spore germination and/or bacterial growth. An exemplary material is a bifidogenic oligosaccharide, which promotes the growth of beneficial probiotic bacteria. In some embodiments, the probiotic bacterial composition is administered with a therapeutically-effective dose of an (preferably, broad spectrum) antibiotic, or an anti-fungal agent. In some embodiments, the compositions described herein are encapsulated into an enterically-coated, time-released capsule or tablet.

The composition may be a food product, such as, but not limited to, a dairy product. The diary product may be cultured or a non-cultured (e.g., milk) dairy product. Non-limiting examples of cultured dairy products include yogurt, cottage cheese, sour cream, kefir, buttermilk, etc. Dairy products also often contain various specialty dairy ingredients, e.g. whey, non-fat dry milk, whey protein concentrate solids, etc. The dairy product may be processed in any way known in the art to achieve desirable qualities such as flavor, thickening power, nutrition, specific microorganisms and other properties such as mold growth control. The compositions of the present invention may also include known antioxidants, buffering agents, and other agents such as coloring agents, flavorings, vitamins, or minerals.

In some embodiments, the compositions of the present invention are combined with a carrier (e.g., a pharmaceutically acceptable carrier) which is physiologically compatible with the gastrointestinal tissue of the subject(s) to which it is administered. Carriers can be comprised of solid-based, dry materials for formulation into tablet, capsule or powdered form; or the carrier can be comprised of liquid or gel-based materials for formulations into liquid or gel forms. The specific type of carrier, as well as the final formulation depends, in part, upon the selected route(s) of administration. The therapeutic composition of the present invention may also include a variety of carriers and/or binders. In some embodiments, the carrier is micro-crystalline cellulose (MCC) added in an amount sufficient to complete the one gram dosage total weight. Carriers can be solid-based dry materials for formulations in tablet, capsule or powdered form, and can be liquid or gel-based materials for formulations in liquid or gel forms, which forms depend, in part, upon the routes of administration. Typical carriers for dry formulations include, but are not limited to: trehalose, malto-dextrin, rice flour, microcrystalline cellulose (MCC) magnesium sterate, inositol, FOS, GOS, dextrose, sucrose, and like carriers. Suitable liquid or gel-based carriers include but are not limited to: water and physiological salt solutions; urea; alcohols and derivatives (e.g., methanol, ethanol, propanol, butanol); glycols (e.g., ethylene glycol, propylene glycol, and the like). Preferably, water-based carriers possess a neutral pH value (i.e., pH 7.0). Other carriers or agents for administering the compositions described herein are known in the art, e.g., in U.S. Patent No. 6,461,607.

### A. Vehicles, Solvents, and Diluents

A composition may comprise a vehicle, solvent, and/or dilutent. Suitable topical vehicles and vehicle components for use with the formulations of the invention are well known in the cosmetic and pharmaceutical arts, and include such vehicles (or vehicle components) as water; organic solvents such as alcohols (particularly lower alcohols readily capable of evaporating from the skin such as ethanol), glycols (such as propylene glycol, butylene glycol, and glycerol (glycerin)), aliphatic alcohols (such as lanolin); mixtures of water and organic solvents (such as water and alcohol), and mixtures of organic solvents such as alcohol and glycerol (optionally also with water); lipid-based materials such as fatty acids, acylglycerols (including oils, such as mineral oil, and fats of natural or synthetic origin), phosphoglycerides, sphingolipids and waxes; protein-based materials such as collagen and gelatin; silicone-based materials (both non-volatile and volatile) such as cyclomethicone, dimethiconol, dimethicone, and dimethicone copolyol; hydrocarbon-based materials such as petrolatum and squalane; and other vehicles and vehicle components that are suitable for administration to the skin, as well as mixtures of topical vehicle components as identified above or otherwise known to the art.

In one embodiment, the compositions of the present invention are oil-in-water emulsions. Liquids suitable for use in formulating compositions of the present invention include water, and water-miscible solvents such as glycols (e.g., ethylene glycol, butylene glycol, isoprene glycol, propylene glycol), glycerol, liquid polyols, dimethyl sulfoxide, and isopropyl alcohol. One or more aqueous vehicles may be present.

In one embodiment, formulations without methanol, ethanol, propanols, or butanols are desirable.

### B. Surfactants and Emulsifiers

A composition may comprise a surfactant and/or emulsifier. Many topical formulations contain chemical emulsions which use surface active ingredients (emulsifiers and surfactants) to disperse dissimilar chemicals in a particular solvent system. For example, most lipid-like (oily or fatty) or lipophilic ingredients do not uniformly disperse in aqueous solvents unless they are first combined with emulsifiers, which form microscopic aqueous soluble structures (droplets) that contain a lipophilic interior and a hydrophilic exterior, resulting in an oil-in-water emulsion. In order to be soluble in aqueous media, a molecule must be polar or charged so as to favorably interact with water molecules, which are also polar. Similarly, to dissolve an aqueous-soluble polar or charged ingredient in a largely lipid or oil-based solvent, an emulsifier is typically used which forms stable structures that contain the hydrophilic components in the interior of the structure while the exterior is lipophilic so that it can dissolve in the lipophilic solvent to form a water-in-oil emulsion. It is well known that such emulsions can be destabilized by the addition of salts or other charged ingredients which can interact with the polar or charged portions of the emulsifier within an emulsion droplet. Emulsion destabilization results in the aqueous and lipophilic ingredients separating into two layers, potentially destroying the commercial value of a topical product.

Surfactants suitable for use in the present invention may be ionic or non-ionic. These include, but are not limited to: cetyl alcohol, polysorbates (Polysorbate 20, Polysorbate 40, Polysorbate 60, Polysorbate 80), steareth-10 (Brij 76), sodium dodecyl sulfate (sodium lauryl sulfate), lauryl dimethyl amine oxide, cetyltrimethylammonium bromide (CTAB), polyethoxylated alcohols, polyoxyethylene sorbitan, octoxynol, N,N-dimethyldodecylamine-N-oxide, hexadecyltrimethylammonium bromide (HTAB), polyoxyl 10 lauryl ether, bile salts (such as sodium deoxycholate or sodium cholate), polyoxyl castor oil, nonylphenol ethoxylate, cyclodextrins, lecithin, dimethicone copolyol, lauramide DEA, cocamide DEA, cocamide MEA, oleyl betaine, cocamidopropyl betaine, cocamidopropyl phosphatidyl PG-dimonium chloride, dicetyl phosphate (dihexadecyl phosphate), ceteareth-10 phosphate, methylbenzethonium chloride, dicetyl phosphate, ceteth-10 phosphate (ceteth-10 is the polyethylene glycol ether of cetyl alcohol where n has an average value of 10; ceteth-10 phosphate is a mixture of phosphoric acid esters of ceteth-10), ceteth-20, Brij S10 (polyethylene glycol octadecyl ether, average Mₙ ∼ 711), and Poloxamers (including, but not limited to, Poloxamer 188 (HO(C₂H₄O)ₐ(CH(CH₃)CH₂O)_{b}(C₂H₄O)ₐH, average molecular weight 8400) and Poloxamer 407 (HO(C₂H₄O)ₐ(CH(CH₃)CH₂O)_{b}(C₂H₄O)ₐH, wherein a is about 101 and b is about 56)). Appropriate combinations or mixtures of such surfactants may also be used according to the present invention.

Many of these surfactants may also serve as emulsifiers in formulations of the present invention.

Other suitable emulsifiers for use in the formulations of the present invention include, but are not limited to, behentrimonium methosulfate-cetearyl alcohol, non-ionic emulsifiers like emulsifying wax, polyoxyethylene oleyl ether, PEG-40 stearate, cetostearyl alcohol (cetearyl alcohol), ceteareth-12, ceteareth-20, ceteareth-30, ceteareth alcohol, Ceteth-20 (Ceteth-20 is the polyethylene glycol ether of cetyl alcohol where n has an average value of 20), oleic acid, oleyl alcohol, glyceryl stearate, PEG-75 stearate, PEG-100 stearate, and PEG-100 stearate, ceramide 2, ceramide 3, stearic acid, cholesterol, steareth-2, and steareth-20, or combinations/mixtures thereof, as well as cationic emulsifiers like stearamidopropyl dimethylamine and behentrimonium methosulfate, or combinations/mixtures thereof.

### C. Moisturizers, Emollients, and Humectants

A composition may comprise a moisturizer, emollient, and/or humectant. One of the most important aspects of topical products in general, and cosmetic products in particular, is the consumer's perception of the aesthetic qualities of a product. For example, while white petrolatum is an excellent moisturizer and skin protectant, it is rarely used alone, especially on the face, because it is greasy, sticky, does not rub easily into the skin and may soil clothing. Consumers highly value products which are aesthetically elegant and have an acceptable tactile feel and performance on their skin.

Suitable moisturizers for use in the formulations of the present invention include, but are not limited to, lactic acid and other hydroxy acids and their salts, glycerol, propylene glycol, butylene glycol, sodium PCA, sodium hyaluronate, Carbowax 200, Carbowax 400, and Carbowax 800.

Suitable emollients or humectants for use in the formulations of the present invention include, but are not limited to, panthenol, cetyl palmitate, glycerol (glycerin), PPG-15 stearyl ether, lanolin alcohol, lanolin, lanolin derivatives, cholesterol, petrolatum, isostearyl neopentanoate, octyl stearate, mineral oil, isocetyl stearate, myristyl myristate, octyl dodecanol, 2-ethylhexyl palmitate (octyl palmitate), dimethicone, phenyl trimethicone, cyclomethicone, C₁₂-C₁₅ alkyl benzoates, dimethiconol, propylene glycol, *Theobroma grandiflorum* seed butter, ceramides (*e.g.,* ceramide 2 or ceramide 3), hydroxypropyl bispalinitamide MEA, hydroxypropyl bislauramide MEA, hydroxypropyl bisisostearamide MEA, 1,3-bis(N-2-(hydroxyethyl)stearoylamino)-2-hydroxy propane, bis-hydroxyethyl tocopherylsuccinoylamido hydroxypropane, urea, aloe, allantoin, glycyrrhetinic acid, safflower oil, oleyl alcohol, oleic acid, stearic acid, dicaprylate/dicaprate, diethyl sebacate, isostearyl alcohol, pentylene glycol, isononyl isononanoate, and 1,3-bis(N-2-(hydroxyethyl)palmitoylamino)-2-hydroxypropane.

In addition, appropriate combinations and mixtures of any of these moisturizing agents and emollients may be used in accordance with the present invention.

### D. Preservatives and Antioxidants

A composition may comprise components adapted to improve the stability or effectiveness of the applied formulation, such as a preservative and/or antioxidant. Suitable preservatives for use in the present invention include, but are not limited to: ureas, such as imidazolidinyl urea and diazolidinyl urea; phenoxyethanol; sodium methyl paraben, methylparaben, ethylparaben, and propylparaben; potassium sorbate; sodium benzoate; sorbic acid; benzoic acid; formaldehyde; citric acid; sodium citrate; chlorine dioxide; quaternary ammonium compounds, such as benzalkonium chloride, benzethonium chloride, cetrimide, dequalinium chloride, and cetylpyridinium chloride; mercurial agents, such as phenylmercuric nitrate, phenylmercuric acetate, and thimerosal; piroctone olamine; *Vitis vinifera* seed oil; and alcoholic agents, for example, chlorobutanol, dichlorobenzyl alcohol, phenylethyl alcohol, and benzyl alcohol.

Suitable antioxidants include, but are not limited to, ascorbic acid and its esters, sodium bisulfite, butylated hydroxytoluene, butylated hydroxyanisole, tocopherols, tocopheryl acetate, sodium ascorbate/ascorbic acid, ascorbyl palmitate, propyl gallate, and chelating agents like EDTA (e.g., disodium EDTA), citric acid, and sodium citrate.

In certain embodiments, the antioxidant or preservative comprises (3-(4-chlorophenoxy)-2-hydroxypropyl)carbamate.

In certain embodiments, antioxidants or preservatives of the present invention may also function as a moisturizer or emollient, for example.

In addition, combinations or mixtures of these preservatives or anti-oxidants may also be used in the formulations of the present invention.

### E. Combination agents

A composition can also contain any other agent that has a desired effect when applied topically to a mammal, particularly a human. Suitable classes of active agents include, but are not limited to, antibiotic agents, antimicrobial agents, anti-acne agents, antibacterial agents, antifungal agents, antiviral agents, steroidal anti-inflammatory agents, non-steroidal anti-inflammatory agents, anesthetic agents, antipruriginous agents, antiprotozoal agents, anti-oxidants, antihistamines, vitamins, and hormones. Mixtures of any of these active agents may also be employed. Additionally, dermatologically-acceptable salts and esters of any of these agents may be employed.

It is known that antimicrobials such as antibiotics can be bactericidal or bacteriostatic, limiting and reducing the quantity of bacteria in our bodies. Retinoids also reduce the amount of bacteria in pilosebacous units and microcomedones. Light based therapies work via photothermal heating, photochemical inactivation of bacteria, and various photoimmunological reactions to improve clinical skin outcomes. In general, the above therapies directly act to reduce the amount of pathogenic bacteria in a patient. Thus, the invention proposes that any such therapy that achieves the same goal of reducing the number of pathogenic organisms, when used in combination with subsequent topical probiotic treatment, would lead to replacement of the pathogenic microflora involved in the diseased state with natural microflora enriched in healthy skin or mucous membranes, or less pathogenic species occupying the same ecological niche as the type causing a disease state.

Suitable antibacterial compounds include capreomycins, including capreomycin IA, capreomycin IB, capreomycin IIA and capreomycin IIB; carbomycins, including carbomycin A; carumonam; cefaclor, cefadroxil, cefamandole, cefatrizine, cefazedone, cefazolin, cefbuperazone, cefcapene pivoxil, cefclidin, cefdinir, cefditoren, cefime, ceftamet, cefmenoxime, cefmetzole, cefminox, cefodizime, cefonicid, cefoperazone, ceforanide, cefotaxime, cefotetan, cefotiam, cefoxitin, cefpimizole, cefpiramide, cefpirome, cefprozil, cefroxadine, cefsulodin, ceftazidime, cefteram, ceftezole, ceftibuten, ceftiofur, ceftizoxime, ceftriaxone, cefuroxime, cefuzonam, cephalexin, cephalogycin, cephaloridine, cephalosporin C, cephalothin, cephapirin, cephamycins, such as cephamycin C, cephradine, chlortetracycline; chlarithromycin, clindamycin, clometocillin, clomocycline, cloxacillin, cyclacillin, danofloxacin, demeclocyclin, destomycin A, dicloxacillin, dirithromycin, doxycyclin, epicillin, erythromycin A, ethanbutol, fenbenicillin, flomoxef, florfenicol, floxacillin, flumequine, fortimicin A, fortimicin B, forfomycin, foraltadone, fusidic acid, gentamycin, glyconiazide, guamecycline, hetacillin, idarubicin, imipenem, isepamicin, josamycin, kanamycin, leumycins such as leumycin A1, lincomycin, lomefloxacin, loracarbef, lymecycline, meropenam, metampicillin, methacycline, methicillin, mezlocillin, micronomicin, midecamycins such as midecamycin A1, mikamycin, minocycline, mitomycins such as mitomycin C, moxalactam, mupirocin, nafcillin, netilicin, norcardians such as norcardian A, oleandomycin, oxytetracycline, panipenam, pazufloxacin, penamecillin, penicillins such as penicillin G, penicillin N and penicillin O, penillic acid, pentylpenicillin, peplomycin, phenethicillin, pipacyclin, piperacilin, pirlimycin, pivampicillin, pivcefalexin, porfiromycin, propiallin, quinacillin, ribostamycin, rifabutin, rifamide, rifampin, rifamycin SV, rifapentine, rifaximin, ritipenem, rekitamycin, rolitetracycline, rosaramicin, roxithromycin, sancycline, sisomicin, sparfloxacin, spectinomycin, streptozocin, sulbenicillin, sultamicillin, talampicillin, teicoplanin, temocillin, tetracyclin, thostrepton, tiamulin, ticarcillin, tigemonam, tilmicosin, tobramycin, tropospectromycin, trovafloxacin, tylosin, and vancomycin, and analogs, derivatives, pharmaceutically acceptable salts, esters, prodrugs, and protected forms thereof.

Suitable anti-fungal compounds include ketoconazole, miconazole, fluconazole, clotrimazole, undecylenic acid, sertaconazole, terbinafine, butenafine, clioquinol, haloprogin, nystatin, naftifine, tolnaftate, ciclopirox, amphotericin B, or tea tree oil and analogs, derivatives, pharmaceutically acceptable salts, esters, prodrugs, and protected forms thereof.

Suitable antiviral agents include acyclovir, azidouridine, anismoycin, amantadine, bromovinyldeoxusidine, chlorovinyldeoxusidine, cytarabine, delavirdine, didanosine, deoxynojirimycin, dideoxycytidine, dideoxyinosine, dideoxynucleoside, desciclovir, deoxyacyclovir, efavirenz, enviroxime, fiacitabine, foscamet, fialuridine, fluorothymidine, floxuridine, ganciclovir, hypericin, idoxuridine, interferon, interleukin, isethionate, nevirapine, pentamidine, ribavirin, rimantadine, stavudine, sargramostin, suramin, trichosanthin, tribromothymidine, trichlorothymidine, trifluorothymidine, trisodium phosphomonoformate, vidarabine, zidoviridine, zalcitabine and 3-azido-3-deoxythymidine and analogs, derivatives, pharmaceutically acceptable salts, esters, prodrugs, and protected forms thereof.

Other suitable antiviral agents include 2',3'-dideoxyadenosine (ddA), 2',3'-dideoxyguanosine (ddG), 2',3'-dideoxycytidine (ddC), 2',3'-dideoxythymidine (ddT), 2'3'-dideoxy-dideoxythymidine (d4T), 2'-deoxy-3'-thia-cytosine (3TC or lamivudime), 2',3'-dideoxy-2'-fluoroadenosine, 2',3'-dideoxy-2'-fluoroinosine, 2',3'-dideoxy-2'-fluorothymidine, 2',3'-dideoxy-2'-fluorocytosine, 2'3'-dideoxy-2',3'-didehydro-2'-fluorothymidine (Fd4T), 2'3'-dideoxy-2'-beta-fluoroadenosine (F-ddA), 2'3'-dideoxy-2'-beta-fluoro-inosine (F-ddI), and 2',3'-dideoxy-2'-beta-flurocytosine (F-ddC). In some embodiments, the antiviral agent is selected from trisodium phosphomonoformate, ganciclovir, trifluorothymidine, acyclovir, 3'-azido-3'-thymidine (AZT), dideoxyinosine (ddI), and idoxuridine and analogs, derivatives, pharmaceutically acceptable salts, esters, prodrugs, and protected forms thereof.

### F. Buffer Salts

A composition may comprise a buffer or salt. Suitable buffer salts are well-known in the art. Examples of suitable buffer salts include, but are not limited to sodium citrate, citric acid, sodium phosphate monobasic, sodium phosphate dibasic, sodium phosphate tribasic, potassium phosphate monobasic, potassium phosphate dibasic, and potassium phosphate tribasic.

### G. Viscosity Modifiers

A composition may comprise a viscosity modifier. Suitable viscosity adjusting agents (*i.e.,* thickening and thinning agents or viscosity modifying agents) for use in the formulations of the present invention include, but are not limited to, protective colloids or non-ionic gums such as hydroxyethylcellulose, xanthan gum, and sclerotium gum, as well as magnesium aluminum silicate, silica, microcrystalline wax, beeswax, paraffin, and cetyl palmitate. In addition, appropriate combinations or mixtures of these viscosity adjusters may be utilized according to the present invention.

### H. Additional constituents

Additional constituents suitable for incorporation into the emulsions of the present invention include, but are not limited to: skin protectants, adsorbents, demulcents, emollients, moisturizers, sustained release materials, solubilizing agents, skin-penetration agents, skin soothing agents, deodorant agents, antiperspirants, sun screening agents, sunless tanning agents, vitamins, hair conditioning agents, anti-irritants, anti-aging agents, abrasives, absorbents, anti-caking agents, anti-static agents, astringents (*e.g.,* witch hazel, alcohol, and herbal extracts such as chamomile extract), binders/excipients, buffering agents, chelating agents, film forming agents, conditioning agents, opacifying agents, lipids, immunomodulators, and pH adjusters (*e.g.,* citric acid, sodium hydroxide, and sodium phosphate).

For example, lipids normally found in healthy skin (or their functional equivalents) may be incorporated into the emulsions of the present invention. In certain embodiments, the lipid is selected from the group consisting of ceramides, cholesterol, and free fatty acids. Examples of lipids include, but are not limited to, ceramide 1, ceramide 2, ceramide 3, ceramide 4, ceramide 5, ceramide 6, hydroxypropyl bispalmitamide MEA, and hydroxypropyl bislauramide MEA, and combinations thereof.

Examples of peptides that interact with protein structures of the dermal-epidermal junction include palmitoyl dipeptide-5 diaminobutyloyl hydroxythreonine and palmitoyl dipeptide-6 diaminohydroxybutyrate.

Examples of skin soothing agents include, but are not limited to algae extract, mugwort extract, stearyl glycyrrhetinate, bisabolol, allantoin, aloe, avocado oil, green tea extract, hops extract, chamomile extract, colloidal oatmeal, calamine, cucumber extract, and combinations thereof.

In certain embodiments, the compositions comprise bergamot or bergamot oil. Bergamot oil is a natural skin toner and detoxifier. In certain embodiments, it may prevent premature aging of skin and may have excellent effects on oily skin conditions and acne.

Examples of vitamins include, but are not limited to, vitamins A, D, E, K, and combinations thereof. Vitamin analogues are also contemplated; for example, the vitamin D analogues calcipotriene or calcipotriol.

In certain embodiments, the vitamin may be present as tetrahexyldecyl ascorbate. This compound exhibits anti-oxidant activity, inhibiting lipid peroxidation. In certain embodiments, use can mitigate the damaging effects of UV exposure. Studies have shown it to stimulate collagen production as well as clarifying and brightening the skin by inhibiting melanogenesis (the production of pigment) thereby promoting a more even skin tone.

Examples of sunscreens include, but are not limited to, p-aminobenzoic acid, avobenzone, cinoxate, dioxybenzone, homosalate, menthyl anthranilate, octocrylene, octyl methoxycinnamate, octyl salicylate, oxybenzone, padimate O, phenylbenzimidazole sulfonic acid, sulisobenzone, titanium dioxide, trolamine salicylate, zinc oxide, 4-methylbenzylidene camphor, methylene bis-benzotriazolyl tetramethylbutylphenol, bis-ethylhexyloxyphenol methoxyphenyl triazine, terephthalylidene dicamphor sulfonic acid, drometrizole trisiloxane, disodium phenyl dibenzimidazole tetrasulfonate, diethylamino hydroxybenzoyl hexyl benzoate, octyl triazone, diethylhexyl butamido triazone, polysilicone-15, and combinations thereof.

Suitable fragrances and colors may be used in the formulations of the present invention. Examples of fragrances and colors suitable for use in topical products are known in the art.

Suitable immunomodulators include, but are not limited to, tetrachlorodecaoxide, deoxycholic acid, tacrolimus, pimecrolimus, and beta-glucan.

In certain embodiments, palmitoyl-lysyl-valyl-lysine bistrifluoroacetate is added. This peptide stimulates collagen synthesis in human fibroblasts.

Often, one constituent of a composition may accomplish several functions. In one embodiment, the present invention relates to constituents that may act as a lubricant, an emollient, or a skin-penetrating agent. In one embodiment, the multi-functional constituent is socetyl stearate, isopropyl isostearate, isopropyl palmitate, or isopropyl myristate.

### II. METHODS OF PREVENTING OR TREATING A CONDITION IN A SUBJECT

In some aspects, the invention relates to a topical composition comprising an RT1 strain of *Propionibacterium acnes* selected from the group consisting of HL050PA2 and HL025PA1 or an RT2 strain of *Propionibacterium acnes* selected from the group consisting of HL001PA1, HL103PA1 and HL106PA1 in a method of preventing or treating a condition in a subject, comprising administering a composition to the subject. The condition may be a skin condition. Administration is topical administration. In some embodiments, the method may comprise administering a strain expressing, encoding, or harboring *deoR.*

In some embodiments, the skin condition may be acne, skin aging, rosacea, or porphyria cutanea tarda.

The subject may be receiving at least 2 µg of vitamin B₁₂ per day, such as at least 3 µg, 4 µg, 5 µg, 6 µg, 7 µg, 8 µg, 9 µg, or at least 10 µg of vitamin B₁₂ per day. A subject may be receiving about 2 µg to about 10 mg of vitamin B₁₂ per day, such as about 2 µg to about 1 mg of vitamin B₁₂ per day, about 3 µg to about 1 mg, about 4 µg to about 1 mg, about 5 µg to about 1 mg, about 6 µg to about 1 mg, about 7 µg to about 1 mg, about 8 µg to about 1 mg, about 9 µg to about 1 mg, or about 10 µg to about 1 mg of vitamin B₁₂ per day.

In some aspects, the disclosure relates to a method for determining the risk that a subject will develop vitamin B₁₂-induced acne. The method may comprise calculating the porphyrin concentration of a cell suspension comprising *P. acnes* obtained from the subject. The method may comprise identifying a strain of *P. acnes* obtained from the subject. The method may comprise identifying the proportion of *P. acnes* obtained from the subject that belong to a particular ribotype, that comprise a gene encoding *deoR,* or that are otherwise associated with acne or healthy skin.

Calculating porphyrin concentration may comprise measuring a concentration of coproporphyrin III or coproporphyrin I, *e.g.,* in a cell suspension of *P. acnes* obtained from a subject. Calculating porphyrin concentration may comprise measuring an absorbance of light, *e.g*., wherein the light has a wavelength of about 380 nm to about 430 nm, such as about 400 nm, about 405 nm, or about 410 nm. Calculating a porphyrin concentration may comprise measuring the cell suspension density, *e.g.,* to normalize the porphyrin concentration measurement. Measuring the cell suspension density may comprise measuring light transmittance through the cell suspension at a wavelength of about 580 nm to about 620 nm, such as a wavelength of about 590 nm, about 595 nm, about 600 nm, or about 605 nm. The method may or may not comprise culturing *P. acnes* obtained from a subject, *e.g.,* prior to calculating a porphyrin concentration.

A method may comprise diagnosing a subject with an elevated risk of developing vitamin B₁₂-induced acne if the porphyrin concentration of a suspension of *P. acnes* obtained from the subject is higher than about 2 µM, such as higher than about 3 µM. A method may comprise diagnosing a subject with a low risk of developing vitamin B₁₂-induced acne if the porphyrin concentration of a suspension of *P. acnes* obtained from the subject is less than about 2 µM.

The method may comprise identifying a strain of *P. acnes* obtained from the subject, and diagnosing the subject with an elevated risk of developing vitamin B₁₂-induced acne if the strain of *P. acnes* is sensitive to vitamin B₁₂. For example, the subject may be diagnosed as having an elevated risk of developing vitamin B₁₂-induced acne if the strain of *P. acnes* is RT4 or RT5. The subject may be diagnosed as having an elevated risk of developing vitamin B₁₂-induced acne if the strain of *P. acnes* is RT3 strain HL063PA2 or RT8 strain HL082PA1. The subject may be diagnosed as having an elevated risk of developing vitamin B₁₂-induced acne if the strain of *P. acnes* is RT1 strain HL005PA2 or HL083PA1.

The method may comprise identifying a strain of *P. acnes* obtained from the subject and diagnosing the subject with a low risk of developing vitamin B₁₂-induced acne if the strain of *P. acnes* is not sensitive to vitamin B₁₂. For example, a subject may be diagnosed as having a low risk of developing vitamin B₁₂-induced acne if the strain of *P. acnes* is RT2 or RT6. Similarly, a subject may be diagnosed as not having an elevated risk of developing vitamin B₁₂-induced acne if the strain of *P. acnes* is RT2 or RT6. A subject may be diagnosed as not having an elevated risk of developing vitamin B₁₂-induced acne if the strain of *P. acnes* is RT3 (i.e., an RT3 strain that is not HL063PA2), RT8 (i.e., an RT8 strain that is not HL082PA1 or HL053PA2), or RT16. The subject may be diagnosed as not having an elevated risk of developing vitamin B₁₂-induced acne if the strain of *P. acnes* is Type II and Type III. In some embodiments, the subject may be diagnosed with a low risk of developing vitamin B₁₂-induced acne if the subject has one or more strains of *P.acnes,* but the majority of *P.acnes* strains are RT2, RT6, RT3 (i.e., an RT3 strain that is not HL063PA2), RT8 (i.e., an RT8 strain that is not HL082PA1 or HL053PA2), RT16, or Type III.

Identifying a strain of *P. acnes* may comprise sequencing a nucleic acid of the strain of *P. acnes.* For example, identifying a strain of *P*. *acnes* may comprise sequencing a nucleotide sequence encoding the 16S rRNA of the strain, or identifying a strain of *P. acnes* may comprise sequencing a nucleotide sequence encoding all or part of the porphyrin biosynthesis gene operon of the strain.

A method may comprise treating a subject with photodynamic therapy if the subject has an elevated risk of developing vitamin B₁₂-induced acne. A method may comprise treating a subject with a treatment other than photodynamic therapy (e.g., with antibiotics, such as topical or oral antibiotics) if the subject has a low risk of developing vitamin B₁₂-induced acne.

A method may comprise administering vitamin B₁₂ to a subject if the subject is not diagnosed with an elevated risk of developing vitamin B₁₂-induced acne. A method may comprise administering vitamin B₁₂ to a subject if the subject is diagnosed with a low risk of developing vitamin B₁₂-induced acne.

In some aspects, the disclosure relates to a method for treating a skin condition in a subject, comprising treating the subject with photodynamic therapy. For example, the method may comprise treating a subject with photodynamic therapy if a *P. acnes* strain of the subject is either an RT4 strain or an RT5 strain. Similarly, the method may comprise treating a subject with photodynamic therapy if the porphyrin concentration of a cell suspension of *P. acnes* obtained from the subject is above a threshold value, such as above about 1 µM, about 2 µM, about 3 µM, about 4 µM, about 5 µM, or about 6 µM.

A method may comprise treating a subject with a treatment other than photodynamic therapy if no *P. acnes* strain of the subject is either an RT4 strain or an RT5 strain. Similarly, the method may comprise treating a subject with a treatment other than photodynamic therapy if the porphyrin concentration of a cell suspension of *P. acnes* obtained from the subject is lower than a threshold value, such as lower than about 1 µM, about 2 µM, about 3 µM, about 4 µM, about 5 µM, or about 6 µM. The method may comprise not treating a subject with photodynamic therapy if the strain of *P. acnes* is RT2 or RT6. In some embodiments, the subject is not treated with photodynamic therapy if the strain of *P. acnes* is Type III.

The method may comprise calculating the porphyrin concentration of a cell suspension comprising *P. acnes* obtained from the subject as described herein, *e.g.,* with or without culturing the *P. acnes* prior to measuring the porphyrin concentration.

In some aspects, the method comprises reducing the amount of porphyrins on the skin of a subject by administering a composition (e.g., an oral or topical composition disclosed herein) comprising one or more strains of *Propionibacterium acnes* disclosed herein to the subject.

The method may comprise determining whether a subject is at risk for a skin condition, the method by obtaining a skin sample from the subject, optionally isolating bacterial DNA from the skin sample, sequencing the bacterial DNA in the skin sample, and if *deor* transcriptional repressor is not expressed, expressed at low levels, or is not present in the bacterial DNA, the subject is considered at risk for a skin disease.

The invention now being generally described, it will be more readily understood by reference to the following examples which are included merely for purposes of illustration of certain aspects and embodiments of the present invention, and are not intended to limit the invention.

### EXEMPLIFICATION

### MATERIALS AND METHODS

***P. acnes* strains and cultures.** *P. acnes* strains used in this study (Table 1) were isolated from the facial skin of acne patients and healthy individuals as described by Fitz-Gibbon et al. (J Invest Dermatol 133:2152-2160 (2013)). Briefly, each strain was isolated from the content collected from multiple hair follicles on the nose of an acne patient or healthy individual. The sampled skin site of acne patients may or may not have visible acne lesions; therefore, the strains do not necessarily correspond to the diseased or healthy state. Four RT4 and RT5 *P. acnes* strains, HL053PA1, HL045PA1, HL043PA1, and HL043PA2, were selected to represent type IA-2 strains, which were associated with the disease based on a 16S metagenomic study (J Invest Dermatol 133:2152-2160 (2013)). Common to most of the RT4 and RT5 strains, these strains harbor mutations in their 16S and 23S rRNA genes, which confer antibiotic resistance. Three RT2 and RT6 strains, HL001PA1, HL103PA1, and HL042PA3, were selected to represent type II strains that were associated with healthy skin. To confirm the findings, an additional three type II strains, HL110PA3, HL106PA1, and HL050PA2, were also tested. The genome sequences of these ten strains were reported previously (Fitz-Gibbon, J Invest Dermatol 133:2152-2160 (2013); Tomida, MBio 4:e00003-00013 (2013)). For each experiment, 5 mL of reinforced clostridial broth was inoculated with 5 X 10⁵ *P. acnes* cells per mL of culture. Cultures were grown to stationary phase anaerobically at 37°C in a light-protected box. Cultures were supplemented on day 0 with vitamin B₁₂ (10 µg/mL), 5-ALA (0.1 mg/mL), and/or LA (0.1 mg/mL). As controls, *P. acnes* strains were also cultured without supplementation. Three to five independent experiments with at least three replicates per experiment were performed for each strain, except strains HL110PA3, HL106PA1, and HL050PA2, which were tested in only one experiment with three replicates.

**Table 1. P. acnes strains**

| | Lineage^{a} | Phylogroup^{b} | | Strain Ribptypr^{c} | Clonal complex (Sequence type by Belfast MLST₈)^{d} | Clonal complex (Sequence type by Aarhus MLST₉)^{e} |
|---|---|---|---|---|---|---|
| **Acne-associated** | IA-2 | IA₁ | HL053PA1 | RT4 | CC3 (ST3) | CC3 (ST3) |
| | | | HL045PA1 | RT4 | CC3 (ST17) | CC3 (ST3) |
| | | | HL043PA1 | RT5 | CC3 (ST3) | CC3 (ST58) |
| | | | HL043PA2 | RT5 | CC3 (ST3) | CC3 (ST58) |
| **Health-associated** | II | II | HL001PA1 | RT2 | CC72 (ST30) | CC60 (ST60) |
| | | | HL103PA1 | RT2 | CC6 (ST25) | CC60 (ST60) |
| | | | HL042PA3 | RT6 | CC6 (ST7) | CC60 (ST73) |
| **Type I with *deoR*** | I | IA₁ | HL025PA1 | RT1 | CC4 (ST4) | CC28 (ST27) |

| | | | | | | |
|---|---|---|---|---|---|---|
| ^{a,c} as described by Fitz-Gibbon et al. (J Invest Dermatol 133:2152-2160 (2013)) ^{b,d} as described by McDowell et al. (J Clin Microbiol 43:326-334 (2005)) ^{e} as described by Lomholt and Kilian (PloS One 5:312277 (2010)). | | | | | | |

**Extraction, identification, and quantification of extracellular porphyrins.** For each strain, porphyrins were extracted using the method described by Kang et al. (Sci Transl Med 7:293ra103 (2015)). Briefly, 500 µL of bacterial culture was extracted in ethyl acetate and acetic acid (4:1, v/v), and solubilized in 1.5M HCl. The absorbance at 405 nm was measured from 200 µL of the soluble phase using a Tecan Genios Spectrophotometer M1000 (Tecan US Inc, Mornsville, NC). The standard curve to convert absorbance to concentration was generated using coproporphyrin III standards of known concentration (C654-3, Frontier Sci). Porphyrin levels were measured in *P. acnes* strain KPA171202 using the quantification method described by Wollenberg et al. (MBio 5:e01286-01214 (2014)). The measurement, which was 206 pmol/mg, is consistent with the reported value of 220 pmol/mg by Wollenberg *et al.* This indicates that the method is comparable to previous studies. Porphyrins were extracted from cultures at stationary phase. The results from cultures at mid-log phase displayed a consistent trend found in cultures at stationary phase. Bacterial culture density was measured at OD₅₉₅ for normalization of porphyrin levels. For mass spectrometry experiments, extracted porphyrins were directly injected onto an Agilent 6460 Triple Quad LC/MS system and each strain's m/z in the negative ion mode was identified.

**Statistical analysis for porphyrin production comparisons.** The average amount of porphyrins produced by each strain under each culture condition was calculated based on the data from at least three independent experiments, with at least three replicates for each. The porphyrin levels between strains, groups (acne-associated vs. health-associated), and culture conditions (with treatment vs. without treatment) were estimated in a linear mixed effect model, with random intercepts by trial to account for trial effects. *P*-values for specific comparisons among groups were corrected using Tukey's method. All statistical analysis was performed using R software (version 3.1.3).

**Identification of *deoR* transcription repressor.** The sequences of the porphyrin gene clusters from 82 *P. acnes* genomes (Tomida, MBio 4:e00003-00013 (2013)) were aligned using the multiple sequence alignment software (ClustalW2). The *deoR* transcription repressor (PPA0299) was identified as an extra genomic element found in all health-associated type II strains and a few type I strains.

**RNA extraction and cDNA synthesis.** For *cbiL* and *deoR* gene expression analysis, cells were lysed with bead beating. Total RNA was extracted using the standard phenolchloroform method and purified using RNeasy kit (Qiagen). DNA was removed using the Turbo DNA-free kit (Life Technologies). RNA quality was assessed using gel electrophoresis. Single-stranded complementary DNA (cDNA) was synthesized using SuperScript III First-Strand Synthesis SuperMix (Life Technologies).

**Analysis of *cbiL* gene expression.** qRT-PCR was performed using the LightCycler 480 High Resolution Melting Master Mix (Roche) on a LightCycler 480 (Roche) with the following primers: cbiL-forward, 5'-GCGCGAGGCAGACGTGATCC-3', and *cbiL*-reverse, 5'-GACACCGGACCTCTCCCGCA-3'. The following qRT-PCR protocol was used: initial denaturation at 95°C for 5 min, followed by 50 cycles of 95°C for 10 sec, 62°C for 30 sec, and 72°C for 30 sec. The fold change in *cbiL* gene expression between cultures with vitamin B₁₂ supplementation and cultures without supplementation was calculated. The gene expression level of *cbiL* in each sample was normalized against the 16S rRNA transcript level. Melting curve analysis was performed using the Light Cycler 480 software Version 1.5 (Roche) to verify the specificity of the amplified products based on their melting temperatures. All reactions were run in triplicate.

**Analysis of *deoR* gene expression.** *deoR* was amplified from the cDNA and genomic DNA of several *P. acnes* strains using a C1000 Thermal Cycler (BioRad). The following primers were used in the PCR: *deoR*-forward, 5'-CTGGCACGAGAAGGAACAA-3', and *deoR-*reverse, 5'-GAATCGAGCAGAACTAGGTCAC-3'. The following PCR protocol was used: initial denaturation at 95°C for 5 min, followed by 35 cycles of 95°C for 10 sec, 62°C for 30 sec, 72°C for 30 sec, followed by one cycle at 72°C for 5 min. Amplified *deoR* products were visualized on a 2% agarose gel. The amplification of 16S rRNA was included as a positive control. Acne-associated strain HL045PA1 was used as a negative control for *deoR* expression.

### RESULTS

**Acne-associated *P. acnes* strains produced significantly more porphyrins than health-associated strains.** To investigate whether different *P. acnes* strains produce the same porphyrin species, the types of porphyrins secreted by multiple *P. acnes* strains where characterized using mass spectrometry. Four acne-associated type IA-2 strains, HL053PA1 (RT4), HL045PA1 (RT4), HL043PA1 (RT5), and HL043PA2 (RT5), and three health-associated type II strains, HL001PA1 (RT2), HL103PA1 (RT2), and HL042PA3 (RT6), were examined (Table 1). No difference in the porphyrin species produced by these *P. acnes* strains was observed. Coproporphyrin III was the dominant porphyrin isomer produced by all strains ([M+H] ⁺ = 655.3). Minimal amounts of coproporphyrin I were also detected. Porphyrin levels are measured with the following method. Bacterial cells are collected, and porphyrins are extracted. The concentration of porphyrins is measured spectrophotometrically and normalized to the optical density (also measured spectrophotometrically). Porphyrin levels are therefore measured per bacterial unit.

To determine whether *P. acnes* strains produce different amounts of porphyrins, the secreted porphyrins in the seven *P. acnes* strains were quantified (Fig. 1). The average porphyrin level produced by acne-associated type IA-2 strains was 6.5µM (5.6 - 8.8 µM), which was significantly greater than that produced by health-associated type II strains, 1.1 µM (0 - 2.1 µM, P<0.0001 (Figure 1)). Notably, the RT2 strain HL001PA1 produced no detectable porphyrins in all experiments. This strain belongs to clonal complex 72 (CC72) based on the Belfast MLST8 scheme (Table 1), and the strain was isolated from healthy skin.

**Vitamin B₁₂ supplementation significantly increased porphyrin production in acne-associated strains, but not in health-associated strains.** To investigate whether vitamin B₁₂ modulates *P. acnes* porphyrin production in a strain-specific manner, the levels of porphyrins produced by different *P. acnes* strains were compared with and without vitamin B₁₂ supplementation. Vitamin B₁₂ supplementation led to increased porphyrin production in all tested acne-associated type IA-2 strains (average porphyrin level increasing from 6.5 µM to 9.2 µM), with statistical significance in three of the four tested strains (*P*≤0.02) (Fig. 2). In contrast, vitamin B₁₂ supplementation had no significant effect on porphyrin production in health-associated type II strains (average porphyrin level remaining at 1.1 µM, *P*≥0.77). The porphyrin levels in HL001PA1 remained undetectable. To confirm these results, porphyrin production was measured, with and without the addition of vitamin B12, in three additional type II strains, HL110PA3 (RT6), HL106PA1 (RT2), and HL050PA2 (RT1). These strains produced similarly low levels of porphyrins (1.4 ± 1.2 µM) and the production level was not significantly affected by vitamin B12 supplementation. This data suggests that vitamin B₁₂ modulates porphyrin production in acne-associated type IA-2 strains, but not in health-associated type II strains, indicating a molecular link between *P. acnes* strain composition of the skin microbiota and the observation that vitamin B₁₂ induces acne in a subset of individuals.

**Vitamin B₁₂ supplementation repressed vitamin B₁₂ biosynthesis gene expression.** To determine whether vitamin B₁₂ affects porphyrin production via repression of its own biosynthesis in *P. acnes,* qRT-PCR was performed to measure the expression level of *cbiL,* a gene in the vitamin B₁₂ biosynthesis pathway. The *cbiL* gene encodes precorrin-2 C20-methyltransferase, one key enzyme involved in corrin ring formation in the vitamin B₁₂ biosynthesis pathway. The addition of vitamin B₁₂ to *P. acnes* cultures resulted in the down-regulation of *cbiL* gene expression, with an average fold change of 0.74 in acne-associated type IA-2 strains and 0.24 in health-associated type II strains (Figure 3). The down-regulation of *cbiL* gene expression is consistent with the previous finding that vitamin B₁₂ supplementation repressed the vitamin B₁₂ biosynthesis pathway, leading to increased porphyrin production. The expression of *cbiL* was down-regulated in both type IA-2 and type II strains, despite the observation that the porphyrin production in type II strains was unaffected by vitamin B₁₂ supplementation. This suggests that additional mechanisms are involved in inhibiting porphyrin production in health-associated type II strains.

**Addition of 5-aminolevulinic acid (5-ALA) increased porphyrin production, which was further enhanced by vitamin B₁₂.** The porphyrin and vitamin B₁₂ biosynthesis pathways in *P. acnes* share the same initial enzymatic steps and a common precursor, 5-ALA, the substrate of the rate-limiting enzyme, porphobilinogen synthase. To investigate whether porphyrin production can be promoted by increasing the availability of 5-ALA, the porphyrin production levels of *P. acnes* strains was compared with and without the addition of the substrate. Additionally, the effect of vitamin B₁₂ on porphyrin production was assessed in combination with 5-ALA. Upon substrate addition only, porphyrin production was significantly increased by an average of 2.2 fold in acne-associated type IA-2 strains (*P*<0.0001) and 3.4 fold in health-associated type II strains (*P*≤0.06, except HL001PA1) compared to untreated controls (Fig. 4). HL001PA1 consistently produced no detectable porphyrins, even upon addition of 5-ALA. Supplementation of 5-ALA in combination with vitamin B₁₂ further enhanced porphyrin production in acne-associated type IA-2 strains (2.7 fold increase compared to substrate only) with statistical significance in three of the four tested strains (*P*≤0.04), but not in health-associated type II strains (*P*≥0.94). This is consistent with the above finding that health-associated type II strains do not respond to vitamin B₁₂ in their porphyrin production (Fig. 2). This data suggests that the metabolic influx of substrates influences porphyrin production in *P*. *acnes.* The modulation of porphyrin production by vitamin B₁₂ supplementation is specific to acne-associated type IA-2 strains, but not health-associated type II strains.

**Small molecule inhibitor reduced porphyrin production in *P. acnes* and its inhibition was counteracted by vitamin B₁₂ supplementation.** To further demonstrate that porphyrin production can be modulated at the metabolic level, the effect of a small molecule inhibitor, levulinic acid (LA), was assessed on porphyrin biosynthesis in *P. acnes* strains. Additionally, the effect of vitamin B12 supplementation on porphyrin production was assessed in combination with LA. LA is an analog of 5-ALA and has been shown in *Pseudomonas aeruginosa* to inhibit the enzymatic activity of porphobilinogen synthase, blocking the metabolic influx to the porphyrin and vitamin B12 biosynthesis pathways. A range of concentrations of LA from 0.1 mg/mL to 1.0 mg/mL was assessed and 0.1 mg/mL LA did not significantly affect the bacterial growth, thus making the measurements of porphyrin production experimentally feasible. LA (0.1 mg/mL) significantly reduced porphyrin production in all strains except HL001PA1 with an average of 30% reduction compared to untreated cultures (*P*≤0.0001) (Fig. 5). However, in acne-associated type IA-2 strains, there was no significant reduction of porphyrins when supplemented in combination with 10 µg/mL vitamin B₁₂. Vitamin B₁₂ supplementation counteracted the inhibition of porphyrin biosynthesis by LA specifically in acne-associated type IA-2strains, but not in health-associated type II strains. This is consistent with our above findings (Figure 2 and Figure 4) and further supports the conclusions that vitamin B₁₂ modulates porphyrin production in acne-associated type IA-2 strains and that porphyrin production can be modulated at the metabolic level. This data also suggests that LA is an effective inhibitor of porphyrin biosynthesis in *P. acnes* and that the porphyrin biosynthesis pathway can be a potential therapeutic target for new acne treatments.

**Health-associated strains harbor a porphyrin biosynthesis repressor gene, *deoR.*** To identify potential molecular mechanisms that can explain differences in porphyrin levels produced by acne-and health-associated *P. acnes* strains, the porphyrin biosynthesis gene operon (*Hem*) of 82 *P. acnes* strains were compared, including the strains tested in this study. All health-associated type II strains and a few type I strains (mainly IB-3 and IC strains) harbor an additional gene in the porphyrin biosynthesis operon, annotated as *deoR* transcriptional repressor (Figures 8 and 9). The *deoR* gene is located 13 bp upstream of the porphyrin biosynthesis gene cluster. This gene is absent in all acne-associated type IA-2 strains. To determine whether *deoR* is functional, gene expression analysis was performed and found to be expressed in two of the three health-associated type II strains, HL001PA1 and HL042PA3, but not in HL103PA1 (Figure 6). Characterizations of *deoR*-like repressors in multiple other species have revealed their roles in regulating cellular processes such as sugar phosphotransferase activity, daptomycin production, and fatty acid beta-oxidation. The close proximity of *deor* to the porphyrin biosynthesis genes in the genome and the presence and expression **of *deoR*** in health-associated type II strains suggest that *deoR* may function as a transcriptional repressor in porphyrin biosynthesis. This may partly explain the low porphyrin levels produced by health-associated type II strains and their associations with healthy skin.

Acne-associated type IA-2strains and health-associated type II strains belong to distinct *P. acnes* lineages. To further investigate whether the low levels of porphyrins produced by health-associated type II strains were potentially due to repression by *deoR* and not due to other lineage differences, the porphyrin levels produced by a type I *P. acnes* strain, HL025PA1 were examined. HL025PA1 represents a distinct lineage within type I and is phylogenetically more similar to type IA-2strains than to type II strains (Figure 9). Unique from most other type I strains, HL025PA1 encodes and expresses *deoR* in its porphyrin biosynthesis operon (Figure 6). HL025PA1 produced porphyrins at a level similar to that of health-associated type II strains (2.7 µM, *P*=0.31), significantly lower than acne-associated type IA-2 strains (*P*=0.0006) (Figure 10). Consistent with the trend observed in health-associated type II strains, vitamin B₁₂ supplementation resulted in its own transcriptional repression in HL025PA1 (Figure 3), while its the porphyrin production was unaffected by vitamin B₁₂ supplementation (Figure 10). This finding further supports an inhibitory role for *deoR* in porphyrin biosynthesis and may potentially explain the health association of type II strains.

## Claims

1. A topical composition comprising an RT1 strain of *Propionibacterium acnes* selected from the group consisting of HL050PA2 and HL025PA1 or an RT2 strain of *Propionibacterium acnes* selected from the group consisting of HL001PA1, HL103PA1 and HL106PA1 for use as a medicament.

2. The topical composition for use of claim 1, wherein the *Propionibacterium acnes* is an RT1 strain selected from the group consisting of HL050PA2 and HL025PA1.

3. The topical composition for use of claim 1, wherein the *Propionibacterium acnes is* an RT2 strain selected from the group consisting of HL001PA1, HL103PA1 and HL106PA1.

4. The topical composition for use of any of claims 1-3, comprising levulinic acid, 4,6-dioxoheptanoic acid, or isonicotinic acid hydrazide.

5. The topical composition for use of any of claims 1-4, wherein the composition is substantially free of RT4 and RT5 strains of *Propionibacterium acnes.*

6. The topical composition for use of any of claims 1-5, wherein the *Propionibacterium acnes* comprises a nucleic acid encoding *deoR.*

7. The topical composition for use of any one of the preceding claims, comprising a retinoid, an antibacterial, a lipid, or a buffering agent.

8. The topical composition for use of any one of the preceding claims, comprising a ceramide.

9. The topical composition for use of any one of the preceding claims, comprising glycerol.

10. The topical composition of any one of the preceding claims for use in the treatment of a skin condition selected from the group consisting of acne, skin aging, rosacea, and porphyria cutanea tarda.

11. A topical composition comprising an RT1 strain of *Propionibacterium acnes* selected from the group consisting of HL050PA2 and HL025PA1 or an RT2 strain of *Propionibacterium acnes* selected from the group consisting of HLOOLPA1, HL103PA1 and HL106PA1 for use in treating a skin condition selected from the group consisting of acne, rosacea, and porphyria cutanea tarda.

12. A cosmetic method of preventing skin aging in a subject comprising administering a topical composition comprising an RT1 strain of *Propionibacterium acnes* selected from the group consisting of HL050PA2 and HL025PA1 or an RT2 strain of *Propionibacterium acnes* selected from the group consisting of HLOOLPA1, HL103PA1 and HL106PA1 to the subject.

## Patentansprüche

1. Topische Zusammensetzung, umfassend einen RT1-Stamm von *Propionibacterium acnes,* ausgewählt aus der Gruppe, bestehend aus HL050PA2 und HL025PA1, oder einen RT2-Stamm von *Propionibacterium* acnes, ausgewählt aus der Gruppe, bestehend aus HL001PA1, HL103PA1 und HL106PA1, zur Verwendung als Medikament.

2. Topische Zusammensetzung zur Verwendung nach Anspruch 1, wobei das *Propionibacterium acnes* ein RT1-Stamm ist, ausgewählt aus der Gruppe, bestehend aus HL050PA2 und HL025PA1.

3. Topische Zusammensetzung zur Verwendung nach Anspruch 1, wobei das *Propionibacterium acnes* ein RT2-Stamm ist, ausgewählt aus der Gruppe, bestehend aus HL001PA1, HL103PA1 und HL106PA1.

4. Topische Zusammensetzung zur Verwendung nach einem der Ansprüche 1 bis 3, umfassend Levulinsäure, 4,6-Dioxoheptansäure oder Isonicotinsäurehydrazid.

5. Topische Zusammensetzung zur Verwendung nach einem der Ansprüche 1 bis 4, wobei die Zusammensetzung im Wesentlichen frei von RT4- und RT5-Stämmen von *Propionibacterium acnes* ist.

6. Topische Zusammensetzung zur Verwendung nach einem der Ansprüche 1 bis 5, wobei das *Propionibacterium acnes* eine Nukleinsäure umfasst, die *deoR* kodiert.

7. Topische Zusammensetzung zur Verwendung nach einem der vorhergehenden Ansprüche, umfassend ein Retinoid, ein antibakterielles Mittel, ein Lipid oder ein Puffermittel.

8. Topische Zusammensetzung zur Verwendung nach einem der vorhergehenden Ansprüche, umfassend ein Ceramid.

9. Topische Zusammensetzung zur Verwendung nach einem der vorhergehenden Ansprüche, umfassend Glycerin.

10. Topische Zusammensetzung nach einem der vorhergehenden Ansprüche zur Verwendung bei der Behandlung eines Hautzustands, ausgewählt aus der Gruppe, bestehend aus Akne, Hautalterung, Rosazea und Porphyria cutanea tarda.

11. Topische Zusammensetzung, umfassend einen RT1-Stamm von *Propionibacterium acnes,* ausgewählt aus der Gruppe, bestehend aus HL050PA2 und HL025PA1, oder einen RT2-Stamm von *Propionibacterium* acnes, ausgewählt aus der Gruppe, bestehend aus HL001PA1, HL103PA1 und HL106PA1, zur Verwendung bei der Behandlung eines Hautzustands, ausgewählt aus der Gruppe, bestehend aus Akne, Rosazea und Porphyria cutanea tarda.

12. Kosmetisches Verfahren zur Verhinderung der Hautalterung bei einem Patienten, umfassend die Verabreichung einer topischen Zusammensetzung, umfassend einen RT1-Stamm von *Propionibacterium acnes,* ausgewählt aus der Gruppe, bestehend aus HL050PA2 und HL025PA1, oder einen RT2-Stamm von *Propionibacterium acnes,* ausgewählt aus der Gruppe, bestehend aus HL001PA1, HL103PA1 und HL106PA1, an den Patienten.

## Revendications

1. Composition topique comprenant une souche RT1 de *Propionibacterium acnes* sélectionnée parmi le groupe constitué de HL050PA2 et HL025PA1 ou une souche RT2 de *Propionibacterium acnes* sélectionnée parmi le groupe constitué de HL001PA1, HL103PA1 et HL106PA1 destinée à être utilisée comme un médicament.

2. Composition topique destinée à être utilisée selon la revendication 1, dans laquelle la *Propionibacterium acnes* est une souche RT1 sélectionnée parmi le groupe constitué de HL050PA2 et HL025PA1.

3. Composition topique destinée à être utilisée selon la revendication 1, dans laquelle la *Propionibacterium acnes* est une souche RT2 sélectionnée parmi le groupe constitué de HL001PA1, HL103PA1 et HL106PA1.

4. Composition topique destinée à être utilisée selon l'une quelconque des revendications 1 à 3, comprenant de l'acide lévulinique, de l'acide 4,6-dioxoheptanoïque ou de l'hydrazide d'acide isonicotinique.

5. Composition topique destinée à être utilisée selon l'une quelconque des revendications 1 à 4, dans laquelle la composition est essentiellement exempte de souches RT4 et RT5 de *Propionibacterium acnes.*

6. Composition topique destinée à être utilisée selon l'une quelconque des revendications 1 à 5, dans laquelle la *Propionibacterium acnes* comprend un acide nucléique codant pour *deoR.*

7. Composition topique destinée à être utilisée selon l'une quelconque des revendications précédentes, comprenant un rétinoïde, un antibactérien, un lipide ou un agent tampon.

8. Composition topique destinée à être utilisée selon l'une quelconque des revendications précédentes, comprenant un céramide.

9. Composition topique destinée à être utilisée selon l'une quelconque des revendications précédentes, comprenant du glycérol.

10. Composition topique selon l'une quelconque des revendications précédentes destinée à être utilisée dans le traitement d'une affection cutanée sélectionnée parmi le groupe constitué de l'acné, du vieillissement cutané, de la rosacée et de la porphyrie cutanée tardive.

11. Composition topique comprenant une souche RT1 de *Propionibacterium acnes* sélectionnée parmi le groupe constitué de HL050PA2 et HL025PA1 ou une souche RT2 de *Propionibacterium acnes* sélectionnée parmi le groupe constitué de HL001PA1, HL103PA1 et HL106PA1 destinée à être utilisée dans le traitement d'une affection cutanée sélectionnée parmi le groupe constitué de l'acné, de la rosacée et de la porphyrie cutanée tardive.

12. Procédé cosmétique de prévention du vieillissement cutané chez un sujet comprenant l'administration d'une composition topique comprenant une souche RT1 de *Propionibacterium acnes* sélectionnée parmi le groupe constitué de HL050PA2 et HL025PA1 ou une souche RT2 de *Propionibacterium acnes* sélectionnée parmi le groupe constitué de HL001PA1, HL103PA1 et HL106PA1 au sujet.
